(19) Europäisches Patentamt
European Patent Office
Office européen des brevets

(11) Veröffentlichungsnummer: 0 102 543
B1

(12) # EUROPÄISCHE PATENTSCHRIFT

(45) Veröffentlichungstag der Patentschrift: **05.11.86**

(51) Int. Cl.[4]: **C 07 D 405/06,** A 01 N 43/64, A 01 N 43/50 // C07D319/06

(21) Anmeldenummer: **83107785.4**

(22) Anmeldetag: **08.08.83**

(54) **Dioxanylazolderivate, diese enthaltende fungizide Mittel und Verfahren zur Bekämpfung von Pilzen mit diesen Verbindungen.**

(30) Priorität: **24.08.82 DE 3231334**

(43) Veröffentlichungstag der Anmeldung: **14.03.84 Patentblatt 84/11**

(45) Bekanntmachung des Hinweises auf die Patenterteilung: **05.11.86 Patentblatt 86/45**

(84) Benannte Vertragsstaaten: **AT BE CH DE FR GB IT LI NL SE**

(56) Entgegenhaltungen:
**EP-A-0 045 016**
**DE-A-2 943 631**
**US-A-4 259 505**

(73) Patentinhaber: **BASF Aktiengesellschaft, Carl-Bosch- Strasse 38, D-6700 Ludwigshafen (DE)**

(72) Erfinder: **Meyer, Norbert, Dr., Stahlbuehlring 155a, D-6802 Ladenburg (DE)**
Erfinder: **Rentzea, Costin, Dr., Neuenheimer Landstrasse 72, D-6900 Heidelberg (DE)**
Erfinder: **Spiegler, Wolfgang, Dr., Amsterdamer Strasse 16, D-6700 Ludwigshafen (DE)**
Erfinder: **Pommer, Ernst- Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

EP 0 102 543 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft Dioxanylazolderivate, fungizide Mittel, die diese Verbindungen enthalten, sowie Verfahren zur Bekämpfung von phytopathogenen Pilzen mit diesen Verbindungen.

Es ist bekannt, daß das 2,2-Dimethyl-4-(1,2,4-triazol-1-yl)-5-phenylpentan-3-on (DE-OS 26 38 470) eine fungizide Wirksamkeit besitzt.

Es ist ferner aus EP-A-45 016 bekannt, Triazolylalkylpyridylether als Fungizide zu verwenden. Die neuen Verbindungen enthalten jedoch keine Pyridylreste.

Aus DE-A-2 943 631 sind fungizide Azolverbindungen mit einem Dioxanylrest bekannt. Diese Verbindungen enthalten jedoch keine Ketogruppe oder ein Derivat einer Ketogruppe.

Es wurde nun gefunden, daß Verbindungen der Formel I

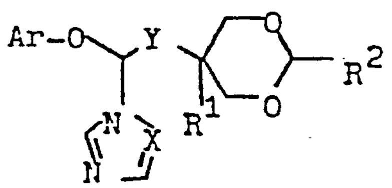

I,

in der

$R^1$ und $R^2$ verschieden oder gleich sind und Wasserstoff oder Alkyl mit 1 bis 5 C-Atomen oder Phenyl bedeuten, wobei der Phenylrest durch Fluor, Chlor, Brom, Nitro, Trifluormethyl, weiterhin durch Alkyl, Alkoxy oder Alkenyl mit jeweils 1 oder 2 bis 4 C-Atomen substituiert sein kann und Ar Bisphenyl, Naphthyl oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 oder 2 bis 5 C-Atomen und ferner durch Phenoxy substituiert sein kann und

Y eine -CO-, -CH(OH)- oder -CH($OR^3$)-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl, ferner durch Alkyl oder Alkoxy mit 1 bis 4 C-Atomen substituiert sein kann, oder $R^3$ einen -CO-$R^4$-Rest bedeutet, wobei $R^4$ einen gegebenenfalls durch Halogen, $C_1$-$C_4$-Alkoxy, Oxo (=O) oder Carb-oxy-$C_1$-$C_4$-alkyl substituierten Alkylrest mit 1 bis 5 C-Atomen oder einen aromatischen Rest bedeutet und X N oder CH bedeutet sowie deren Salze und Metallkomplexverbindungen ausgezeichnet wirksam gegen Schadpilze sind.

Die neuen Verbindungen der Formel I enthalten chirale Zentren und werden im allgemeinen in Form von Racematen oder als Diastereomerengemische von erythro- sowie threo-Formen erhalten. Die erythro- und threo-Diastereomeren lassen sich bei einigen der neuen Verbindungen beispielsweise durch Löslichkeitsunterschiede oder durch Säulenchromatographie trennen und in reiner Form isolieren. Aus solchen einheitlichen Diastereomerenpaaren kann man mit bekannten Methoden einheitliche Enantiomere erhalten. Diese sowie die oben beschriebenen Mischungen werden von der vorliegenden Erfindung umfaßt. Für die Anwendung der neuen Verbindungen sind sowohl die einheitlichen Diastereomeren bzw. Enantiomeren wie auch deren bei der Synthese anfallende Gemische geeignet. Bevorzugt werden die letzten verwendet.

$R^1$ und $R^2$ bedeuten bespielsweise Wasserstoff, Methyl, Ethyl, n-Propyl, Isopropyl, n-Butyl, sek.-Butyl, iso-Butyl, tert.-Butyl, n-Pentyl, Neopentyl, Phenyl, 4-Fluor-phenyl, 2-Chlorphenyl, 3-Chlorphenyl, 4-Chlorphenyl, 4-Bromphenyl, 2,4-Dichlorphenyl, 3,4-Dichlorphenyl, 2-Meth-oxiphenyl, 2,4-Dimethoxiphenyl, 3-Methoxiphenyl, 4-Methoxiphenyl, 4-Ethoxiphenyl, 4-tert.-Butoxiphenyl, 4-Methylphenyl, 4-Ethylphenyl, 4-Isopropylphenyl, 4-tert.-Butyl-phenyl, 3-Nitrophenyl, 4-Nitrophenyl, 3-Trifluormethylphe-nyl, 4-Trifluormethylphenyl.

Ar bedeutet beispielsweise 4-Biphenyl, 1- und 2-Naphthyl, Phenyl, 4-Fluorphenyl, 2-Chlorphenyl, 3-Chlorphenyl, 4-Chlorphenyl, 4-Bromphenyl, 2,4-Dichlorphenyl, 3,4-Dichlorphenyl, 3,5-Dichlor-2-Methoxyphenyl, 2,3,4-Tri-chlorphenyl, 2-Methoxyphenyl, 2,4-Dimethoxyphenyl, 3-Methoxy-phenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 3-Nitrophenyl, 4-Nitrophenyl, 3-Trifluor-methylphenyl, 4-Trifluormethylphenyl und 4-Phenoxyphenyl.

X bedeutet beispielsweise eine Carbonylgruppe (C=O) oder die daraus durch Reduktion resultierende Alkoholgruppe (CHOH), die man verethern (zu CH-$OR^3$) oder verestern (zu CH-$OCOR^4$) kann. Dabei bedeutet beispielsweise $R^3$ Methyl, Ethyl, N-Propyl, n-Butyl, 4-Chlorbutyl, n-Pentyl, n-Hexyl, Allyl, Buten-2-yl, Propargyl, Benzyl, 4-Fluorbenzyl, 4-Chlorbenzyl, 4-Brombenzyl, 2,4-Dichlorbenzyl und 4-Trifluormethylbenzyl. $R^4$ bedeutet vorzugsweise Methyl, Ethyl, n-Propyl, Isopropyl, Chlormethyl, Chlorpropyl, Methoxymethyl, Vinyl und Propenyl.

Säureaditionssalze sind z.B. die Salze mit Salzsäure, Schwefelsäure, Bromwasserstoffsäure, Salpetersäure, Phosphorsäure, Oxalsäure, Dodecylbenzolsulfonsäure. Metallkomplexe sind z.B. die Komplexe mit Kupfer-, Zink-, Zinn-, Mangan-, Eisen-, Kobalt-, Nickelsalzen.

Die neuen Verbindungen lassen sich herstellen, indem man

a) 1,2,4-Triazol oder Imidazol mit alpha-Halogenketonen der Formel II

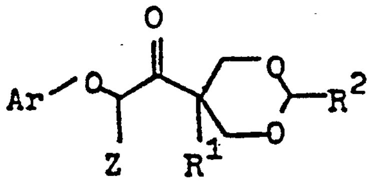

II,

worin $R^1$, $R^2$ und Ar die oben genannte Bedeutung haben und Z für Chlor oder Brom steht oder

b) einen Alkohol der Formel III

Ar-OH III,

worin Ar die oben angegebene Bedeutung hat, mit einem Keton der Formel IV

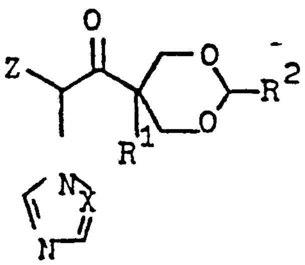

IV,

worin $R^1$, $R^2$, Z und X die oben genannte Bedeutung haben, umsetzt und die so erhaltenen Verbindungen gegebenenfalls reduziert und verethert oder verestert.

Die Reaktion gemäß Verfahrensvariante a) und b) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120° C. Zu den bevorzugten Lösungs- und Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium, Natrium- oder Kaliumhydroxid, Alkalicarbonate wie Natrium- oder Kaliumcarbonat oder Natrium- und Kaliumhydrogencarbonat, Pyridin oder 4-Dimethylaminopyridin. Es können aber auch andere übliche Basen verwendet werden.

Bei der Verfahrensvariante a) kann auch ein entsprechender Überschuß an 1,2,4-Triazol bzw. Imidazol verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyl-triethylammoniumchlorid oder -bromid oder Kronenether wie 12-Krone-4, 15-Krone-5, 18-Krone-6, Diben-zo-18-Krone-6 oder Dicyclohexano-18-Krone-6 in Frage.

Die Chlorketone der Formel II (Z = Cl) können beispielsweise durch Chlorierung von Verbindungen der Formel V

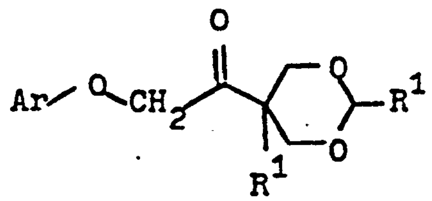

V,

nach allgemein bekannten Verfahren hergestellt werden (z.B. W. Hahn und R. Stroh in Houben-Weyl, Müller, Methoden der organischen Chemie, Georg Thieme Verlag, Stuttgart 1962, Bd 5/3).

Die Aryloxiketone V erhält man aus bekannten alpha-Halogenketonen der Formel VI

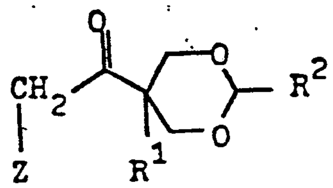

VI,

worin $R^1$, $R^2$ und Z die oben angegebene Bedeutung haben und Alkoholen der Formel III unter den für die Umsetzung von Alkoholen (III) mit Halogenketonen (V) oben beschriebenen Bedingungen.

Die aus der DE-OS 30 25 879 bekannten 2-Halogen-1-(2,5-di-alkyl-1,3-dioxan-5-yl)-ethan-1-one der Formel VI sind auch Vorprodukte für das Verfahren b). Nach dort auch beschriebenen Verfahren werden sie in die alpha-Azolylketone IV überführt. Das noch verbliebene aktive Wasserstoffatom wird anschließend in üblicher Weise gegen Halogen ausgetauscht, um die Verbindungen der Formel IV zu erhalten, die unter den oben angegebenen Bedingungen zu den Ketonen I (Y = CO) umgesetzt werden.

Die gemäß a) oder b) erhaltenen Ketone lassen sich gegebenenfalls in Gegenwart eines Lösungs- oder Verdünnungsmittels, bei Temperaturen zwischen -20 bis 150° C, bei Normaldruck oder unter erhöhtem Druck mit Wasserstoff in Gegenwart eines Katalysators oder mit komplexen Bor- bzw. Aluminiumhydriden, mit Aluminiumisopropoxid und Isopropanol, mit Natriumdithionit oder elektrochemisch reduzieren.

Geeignete Lösungs- oder Verdünnungsmittel hierfür sind beispielsweise Wasser, Methanol, Ethanol, Isopropanol, Essigsäure, Diethylether, Tetrahydrofuran, Dioxan, Essigester, Toluol, Dimethylformamid oder entsprechende Gemische.

Zur katalytischen Hydrierung verwendet man Platin- oder Palladiumkatalysatoren auf inerten Trägern und hydriert bei Drucken von 2 bis 80 bar bis keine Wasserstoffaufnahme mehr erfolgt.

Für die als Reduktionsmittel verwendbaren Hydride seien beispielsweise Natriumborhydrid oder Lithiumaluminiumhydrid genannt.

Die so erhaltenen Alkohole der Formel I (X =

CHOH) lassen sich mit Alkylierungsmitteln der Formel VII

Z-$R^3$ VII,

worin $R^3$ die oben angegebene Bedeutung hat und Z Chlor oder Brom darstellt, gegebenenfalls in Gegenwart eines Lösungs- oder Verdünnungsmittels die Ein- oder Zweiphasensysteme bilden, gegebenenfalls unter Zusatz einer anorganischen Base und gegebenenfalls unter Zusatz eines Reaktionsbeschleunigers und/oder Phasentransferkatalysators verethern.

Hierfür eignen sich folgende Lösungs- oder Verdünnungsmittel: Diethylether, Tetrahydrofuran, Dioxan, n-Pentan, monohalogenierte Kohlenwasserstoffe mit 2 bis 6 C-Atomen wie beispielsweise Chlorethan, Bromethan, 1-Chlorpropan, 1-Brompropan, 1-Chlorbutan, 1-Brombutan, 1-Chlorpentan, 1-Brompentan, 1-Chlorhexan, 1-Bromhexan, ferner Cyclohexan, Methylenchlorid, Chloroform, Toluol oder Dimethylformamid.

Als organische Basen seien beispielsweise genannt: Alkali- und Erdalkalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat, Magnesiumethylat, Magnesiummethylat, Natriumisopropylat oder Kalium-tert.-butylat.

Geeignete Reaktionsbeschleuniger sind beispielsweise Metallhalogenide wie Natriumbromid, Natriumiodid, Kaliumbromid, Kaliumiodid, tertiäre Amine wie 4-Dimethylamino-pyridin und 4-Pyrrolidinopyridin, Kronenether wie 12-Krone-4-, 14-Krone-5, 18-Krone-6, Dibenzo-18-Krone-6 oder Dicyclohexano-18-Krone-6 oder Azole wie Imidazol und 1,2,4-Triazol.

Als Phasentransferkatalysatoren kommen vorzugsweise quaternäre Ammoniumsalze wie Tetrabutylammoniumchlorid, -bromid, -iodid oder -hydrogensulfat, Benzyl-triethylammoniumchlorid, Methyltrioctylammoniumchlorid und -bromid und Phosphoniumsalze wie Tetrabutylphosphoniumbromid und -iodid und Tetra-n-pentylphosphoniumbromid und -iodid in Frage.

Die Veresterung der Alkohole der Formel I (X = CHOH) kann mit Säurechloriden oder -anhydriden der Formeln Y-CO-$R^4$ (VIII) oder $(R^4\text{-}CO)_2O$ (IX) gegebenenfalls in Gegenwart eines Lösungs- oder Verdünnungsmittels, gegebenenfalls unter Zusatz eines säurebindenden Mittels und gegebenenfalls unter Zusatz eines Reaktionsbeschleunigers erfolgen. Hierfür eignen sich dieselben Lösungsmittel und Basen wie für die Veretherung. Zusätzlich können tertiäre Amine wie Trimethylamin, Triethylamin, N,N-Dimethylanilin, N,N-Dimethylcyclohexylamin, N-Methylpiperidin oder Pyridin als Base verwendet werden.

Mit geeigneten Basen wie z.B. Alkalihydrid wie Natriumhydrid oder Alkali- oder Erdalkalialkoholaten wie Natriummethylat können die Alkohole auch in einer ersten Umsetzung zunächst in ihre Alkoholat-Salze überführt werden und dann als solche zur Reaktion gebracht werden.

Die so erhaltenen Verbindungen der Formel I werden nach üblichen Methoden isoliert, gegebenenfalls gereinigt und gegebenenfalls mit Säuren, z.B. Salzsäure, Schwefelsäure, Bromwasserstoffsäure, Salpetersäure, Phosphorsäure, Oxalsäure, Dodecylbenzolsulfonsäure oder Metallsalzen, z.B. Kupfersulfat, Kupferchlorid, Zinkchlorid, Zinnchlorid, Mangansulfat, Eisensulfat, Kobaltsulfat, Kobaltchlorid, Nickelchlorid zu Salzen bzw. zu Metall-Komplexen umgesetzt.

**Beispiel 1**

a) Herstellung der Ausgangsverbindungen

Zu einer Lösung von 144 g (1 Mol) 5-Acetyl-5-methyl-1,3-dioxan und 85,5 g (1 Mol) alpha-Pyrrolidon in 500 ml Tetrahydrofuran wird in 2 Stunden bei 50° C die Lösung von 498 g (1 Mol) alpha-Pyrrolidon-Brom-Komplex in 1 Liter Tetrahydrofuran zugetropft. Nach achtstündigem Rühren bei 50° C wird der weiße Niederschlag von Pyrrolidon-Hydrobromid abgesaugt, mit 50 ml Tetrahydrofuran gewaschen und das Filtrat im Vakuum eingeengt. Man erhält 220 g (99 %) rohes, öliges 1-(5-Methyl-1,3-dioxan-5-yl)-2-brom-ethan-1-on.

Man mischt bei Raumtemperatur 276 g (2 Mol) fein pulverisiertes Kaliumcarbonat, 800 ml trockenes Aceton und 128,6 g (1 Mol) 4-Chlorphenol und tropft unter heftigem Rühren 223 g (1 Mol) 1-(5-Methyl-1,3-dioxan-5-yl)-2-brom-ethan-1-on bei 25° C in 2 Stunden zu. Nach achtstündigem Erhitzen auf 50 - 55° C läßt man abkühlen, saugt ab, wäscht den Rückstand mit Aceton nach und engt das Filtrat ein. Der dabei anfallende Rückstand wird in 1 Liter Methylenchlorid aufgenommen, mit 5%iger Natronlauge und Wasser gewaschen, getrocknet und eingeengt. Durch Digerieren mit Petrolether erhält man 179,8 g (66,5 %) 1-(5-Methyl-1,3-dioxan-5-yl)-2(4-chlorphenoxy)-ethan-1-on. Schmp. 63-66° C.

54 g (0,2 Mol) 1-(5-Methyl-1,3-dioxan-5-yl)-2-(4-chlorphen-oxy)-ethan-1-on werden in 400 ml Tetrachlorkohlenstoff gelöst und auf 60° C erwärmt. Man tropft 29,6 g (0,22 Mol) Sulfurylchlorid, gelöst in 40 ml Tetrachlorkohlenstoff, zu und rührt noch 15 Stunden bei dieser Temperatur nach. Dann wird das Lösungsmittel im Wasserstrahlvakuum abdestilliert und der Rückstand mit Petrolether/Methyl-tert.-butylether (1:1) verrührt, wobei 50,2 g (83,4 %) 1-(5-Methyl-1,3-dioxan-5-yl)-2-chlor-2-(4-chlorphenoxy)-ethan-1-on anfallen. Schmp. 115 bis 117° C.

b) Herstellung des Endproduktes

12,2 g (0,04 Mol) 1-(5-Methyl-1,3-dioxan-5-yl)-2-chlor-2-(4-chlorphenoxy)-ethan-1-on wurden in 100 ml trockenem Acetonitril gelöst und mit 5,52 g (0,08 Mol) 1,2,4-Triazol versetzt. Man ließ 16 Stunden unter Rückfluß kochen, saugte nach dem Abkühlen ab, engte das Filtrat ein, nahm den Rückstand in Methylenchlorid auf und wusch ihn dreimal mit je 50 ml Wasser. Nach Trocknen der organischen Phase über Natriumsulfat und Entfernen des Lösungsmittels erhielt man einen hellen Kristallbrei, der mit Methyl-tert.-butylether verrührt wurde. Dabei kristallisierten 9,3 g (68,9 %) 1-(5-Methyl-1,3-dioxan-5-yl)-2-(4-chlorphenoxy)-2-(1,2,4-triazol-1-yl)-ethan-1-on. Schmp. 98° C (Verbindung Nr. 1).

**Beispiel 2**

Zu einer Lösung von 16,9 g (0,05 Mol) 1-(5-Methyl-1,3-di-oxan-5-yl(-2-(4-chlorphenoxy)-2-(1,2,4-triazol-1-yl)-ethan-1-on in 100 ml Methanol wurde bei Raumtemperatur portionsweise 1,0 g (0,016 Mol) Natriumborhydrid zugegeben. Nach fünfstündigem Rühren engte man ein, nahm in Methylenchlorid auf und wusch die organische Phase mit Wasser neutral. Nach Trocknen über Natriumsulfat und Einengen fielen 14,5 g (85,3 %) 1-(5-Methyl-1,3-dioxan-5-yl)-(2-(4-chlor-phenoxy)-2-(1,2,4- triazol-1-yl)-ethan-1-ol als Diastereomerengemisch mit Schmp. 68 - 80° C an (Verbindung Nr. 2).

**Beispiel 3**

Man löste 18,0 g (0,062 Mol) 1-(5-Methyl-1,3-dioxan-5-yl)-2-chlor-2-(4-fluorphenoxy)-ethan-1-on in 150 ml trockenem Acetonitril und fügte unter Rühren 8,34 g (0,124 Mol) Imidazol hinzu. Nach dreitägigem Stehen bei Raumtemperatur (20° C) wurde vom Ungelösten abgesaugt, das Filtrat eingeengt und dreimal mit Wasser gewaschen. Man trocknete die organische Phase über $Na_2SO_4$ und engte ein. Dabei fiel ein helles Öl an, das in Essigester gelöst und über Kieselgel filtriert wurde. Die Hauptfraktion enthielt 9,9 g (49,9 %) 1-(5-Methyl-1,3-dioxan-5-yl)-2-(4-fluorphenoxy)-2-(imidazo 1-1-yl)-ethan-1-on. Schmp. 115° C (Verbindung Nr. 3).

**Beispiel 4**

5,47 g (0,016 Mol) 1-(5-Methyl-1,3-dioxan-5-yl)-2-(4-Chlor-phenoxy)-2-(1,2,4-triazol-1-yl)-ethan-1-ol, 3,5 ml (0,025 Mol) Triethylamin, 3,3 g (0,025 Mol) Propionsäureanhydrid und 0,13 g (0,0013 Mol) 4-N,N-Dimethylaminopyri-din wurden gemischt und 24 Std. bei Raumtemperatur (20° C) gerührt. Dann nahm man in Essigester auf, wusch zweimal mit je 50 ml 2N Salzsäure und dreimal mit je 50 ml 10%iger Natriumhydrogencarbonatlösung, trocknete die organische Phase über Natriumsulfat und verrührte nach dem Einengen mit Petrolether. Dabei erhielt man 5,3 g (83,7 %) 1-(5-Methyl-1,3-dioxan-5-yl)-1-propionoxi-2-(4-chlorphenoxy-(2-(1,2,4-triazol-1-yl)-ethan als Diastereomerengemisch. Schmp. 99 - 117° C (Verbindung Nr. 4).

Entsprechend wurden diejenigen in der folgenden Tabelle angegebenen Verbindungen der Formel I hergestellt, deren Schmelzpunkte (Fp) oder IR-Banden angegeben sind. Ihre Struktur wurde durch Ultrarot- und Kernresonanz-Spektroskopie oder durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden; es kann erwartet werden, daß sie aufgrund ihrer ähnlichen Konstitution ähnliche Wirkungen wie die näher untersuchten Verbindungen haben.

| Bsp. Nr. | Ar | X | Y | $R^1$ | $R^2$ | Fp[°C],IR(Film)$cm^{-1}$ |
|---|---|---|---|---|---|---|
| 1 | 4-Chlorphenyl | N | CO | Methyl | H | 98 |
| 2 | " | N | CHOH | " | H | 68-80 |
| 3 | 4-Fluorphenyl | CH | CO | " | H | 115 |
| 4 | 4-Chlorphenyl | N | $COOC_2H_5$ | " | H | 99-117 |
| 5 | " | N | CHOH | " | H | .1/2 $CuCl_2$ 164-174 |
| 6 | " | CH | CO | " | H | 2683, 1733, 1593, 1490, 1457, 1212, 1164, 1087, 1030, 1012 |
| 7 | " | N | CO | " | Isopropyl | 1736, 1504, 1491, 1277, 1225, 1196, 1133, 1097, 1009 |
| 8 | 2,4-Dichlorphenyl | N | CHOH | " | H | 3426, 1506, 1479, 1280, 1266, 1238, 1197, 1164, 1132, 1105, 1087, 1062, 1031 |
| 9 | " | N | CO | " | H | 1733, 1506, 1478, 1279, 1233, 1212, 1199, 1181, 1164, 1133, 1106, 1087, 1060, 1032 |
| 10 | 3,4-Dichlorphenyl | N | CO | " | H | 113-115 |
| 11 | " | N | CHOH | " | H | 135-143 |
| 12 | 4-Chlor-2-methylphenyl | N | CO | " | H | 78-84 |
| 13 | " | N | CHOH | " | H | 56-62 |
| 14 | 4-Phenylphenyl | N | CO | " | H | 118-121 |

| Bsp. Nr. | Ar | X | Y | $R^1$ | $R^2$ | Fp[°C],IR(Film)$cm^{-1}$ |
|---|---|---|---|---|---|---|
| 15 | 4-Phenylphenyl | N | CHOH | Methyl | H | 71-80 |
| 16 | " | CH | CO | " | H | 1733, 1480, 1223, 1164, 1086, 1074, 1031, 1011, 927 |
| 17 | 4-Fluorphenyl | N | CO | " | H | 106-108 |
| 18 | " | N | CHOH | " | H | 133-137 |
| 3 | " | CH | CO | " | H | 115 |
| 19 | " | N | CO | " | H | 1/2 $CuCl_2$ 178-181 |
| 20 | 4-Bromphenyl | N | CO | " | H | 87-88 |
| 21 | " | N | CHOH | " | H | 49-54 |
| 22 | " | CH | CO | " | H | 1732, 1487, 1213, 1164, 1087, 1071, 1030, 1007, 926 |
| 23 | 4-Trifluormethylphenyl | N | CO | " | H | 1488, 1462, 1180, 1125, 1093, 1072, 1012 |
| 24 | " | N | CHOH | " | H | 131-141 |
| 25 | " | CH | CO | " | H | 76-78 |
| 26 | 2,6-Dichlorphenyl | N | CO | " | Phenyl | 1736, 1478, 1390, 1277, 1234, 1132, 1094, 1077, 1027 |
| 27 | 4-Chlorphenyl | N | CO | " | n-Butyl | |
| 28 | 3,4-Dichlorphenyl | N | $CHOCH_2CH=CH_2$ | " | H | |
| 29 | 4-Fluorphenyl | N | $CHOCH_2C_6H_5$ | " | H | |

| Bsp. Nr. | Ar | X | Y | $R^1$ | $R^2$ | Fp[°C],IR(Film)$cm^{-1}$ |
|---|---|---|---|---|---|---|
| 30 | 4-Bromphenyl | N | $CHOCH_3$ | Methyl | H | |
| 4 | 4-Chlorphenyl | N | $CHOCOCH_2CH_3$ | " | H | 99-117 |
| 31 | Phenyl | N | CO | " | H | |
| 32 | " | N | CHOH | " | H | |
| 33 | " | CH | CO | " | H | |
| 34 | 4-Chlorphenyl | N | CHOH | " | n-Propyl | |
| 35 | " | N | C=O | " | " | |
| 36 | 2,4-Dichlorphenyl | N | C=O | " | " | |
| 37 | 2,4-Dichlorphenyl | N | CHOH | " | " | |
| 38 | " | CH | CO | " | " | |
| 39 | " | N | CO | " | Isopropyl | |
| 40 | " | N | CHOH | " | " | |
| 41 | " | N | CO | " | n-Butyl | |
| 42 | " | N | CHOH | " | " | |
| 43 | 4-Chlorphenyl | N | CO | Ethyl | Ethyl | |
| 44 | " | N | CHOH | " | " | |
| 45 | " | N | CO | Methyl | 4-Chlorphenyl | |
| 46 | " | N | CHOH | " | " | |
| 47 | " | N | CO | " | 2,4-Dichlor-phenyl | |
| 48 | " | N | CHOH | " | " | |

Die neuen Verbindungen und ihre Salze und Metallkomplexverbindungen zeichnen sich durch eine hervorragende Wirksamkeit gegen ein breites Spektrum von pflanzenpathogener Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u.a. Zur Bekämpfung holzzerstörender Pilze, wie Coniophora puteana und Polystictus versicolor, verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an äpfeln,
Septoria nodorum an Weizen,
Botrytis cinerea (Grauschimmel) an Erdbeeren, Reben,
Phthophthora infestans (falscher Mehltau) an Kartoffeln,
Tomaten,
Pyricularia oryzae an Reis.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol,

Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha.

Die neuen Verbindungen können auch im Materialschutz u.a. zur Bekämpfung holzzerstörender Pilze, wie Coniophora puteana und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 Gew.% Wirkstoff, bezogen auf das Gesamtgewicht der zu konservierenden Farben bzw. des mikrozid auszurüstenden Polyvinylchlorids. Die neuen Wirkstoffe könen auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Verspürhen, Vernebeln, Verstäuben, Verstreuen, Beizen oder Gießen.

I. 20 Gewichtsteile der Verbindung des Beispiels 2 werden in 3 Gewichtsteilen des Natriumsalzes der Diisobutylnaphthalinsulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

II. 3 Gewichtsteile der Verbindung des Beispiels 7 werden mit 97 Gewichtsteilen feinteiligem Kaolin innig vermischt. Man erhält auf diese Weise ein Stäubemittel, das 3 Gew.% des Wirkstoffs enthält.

III. 30 Gewichtsteile der Verbindung des Beispiels 8 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 40 Gewichtsteile der Verbindung des Beispiels 9 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.

V. 20 Teile der Verbindung des Beispiel 11 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung des Beispiels 17 mit 10 Gewichtsteilen N-Methyl-alpha-pyrrolidon und erhält eine Lösung, die zur Anwendung in Form kleinster Tropfen geeignet ist.

VII. 20 Gewichtsteile der Verbindung des Beispiels 18 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

VIII. 20 Gewichtsteile der Verbindung des Beispiels 21 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IX. 10 Gewichtsteile der Verbindung des Beispiels 24, 20 Gewichtsteile Polyoxyethylensorbitan-monolaurat, 20 Gewichtseile Methanol und 50 Gew.-Teile Wasser werden zu einer Lösung verrührt, die 10 Gew.% des Wirkstoffs enthält. Durch Hinzufügen von weiterem Wasser können stärker verdünnte Lösungen hergestellt werden.

Die neuen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des Wirkungsspektrums. Bei einer Anzahl solcher Fungizidmischungen treten auch synergistische Effekte auf, d.h. die Wirksamkeit des Kombinationsproduktes ist größer als die addierten Wirksamkeiten der Einzelkomponenten.

Fungizide, die mit den neuen Verbindungen kombiniert werden können, sind beispielsweise:

Schwefel,

Dithiocarbamate und deren Derivate, wie

Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,
heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-di-methylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicar-bonsäureimid.

Die folgenden Versuche erläutern die fungizide Wirkung. Für diese Versuche wurde als bekannter Vergleichswirkstoff A die Verbindung 2,2-Dimethyl-4-(1,2,4-Triazol-1-yl)-5-phenylpentan-3-on (bekannt aus DE-OS 26 38 470) verwendet.

**Versuch 1**

Wirksamkeit gegen Weizenmehltau

Blätter von in Töpfen gewachsenen Weizenkeimlingen der Sorte "Jubilar" werden mit wäßriger Spritzbrühe, die 80 % Wirkstoff und 20 % Emulgiermittel in der Trockensubstanz enthält, besprüht und 24 Stunden nach dem

Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22° C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß beispielsweise die Verbindungen 2, 7, 8, 9, 11, 17, 18, 21, 24 und 25 bei Anwendung als 0,025; 0,006 oder 0,0015%ige Spritzbrühe (Gew.%) eine bessere fungizide Wirkung (beispielsweise 97 %) zeigten als der Wirkstoff A (beispielsweise 80 %).

**Versuch 2**

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22° C und 70 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß die Verbindungen 1, 2, 5, 7, 8, 9, 10, 11, 12, 13, 14, 15, 17, 18, 19, 20, 21, 24 und 26 bei Anwendung als 0,025%ige Spritzbrühe eine bessere fungizide Wirkung (beispielsweise 97 %) zeigten als der Wirkstoff A (80 %).

**Versuch 3**

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22° C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22° C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß die Verbindungen 3, 6, 7, 15, 16, 18 und 21 bei Anwendung als 0,025%ige Spritzbrühe eine bessere fungizide Wirkung (beispielsweise 97 %) zeigten als der Wirkstoff A (90 %).

**Versuch 4**

Wirksamkeit gegen Botrytis cinerea an Paprika

Paprikasämlinge der Sorte "Neusiedler Ideal Elite" werden, nachdem sich 4 - 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 - 24° C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß die Wirkstoffe 6, 8, 15, 16 und 22 bei Anwendung als 0,05%ige Spritzbrühe eine bessere fungizide Wirkung (beispielsweise 97 %) zeigten als der Wirkstoff A (70 %).

**Patentansprüche**

1. Dioxanyl-azolderivat der Formel I

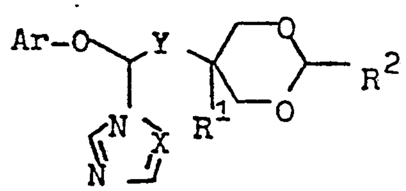 I,

in der

$R^1$ und $R^2$ verschieden oder gleich sind und Wasserstoff oder Alkyl mit 1 bis 5 C-Atomen oder Phenyl bedeuten, wobei der Phenylrest durch Fluor, Chlor, Brom, Nitro, Trifluormethyl, weiterhin durch Alkyl, Alkoxy oder Alkenyl mit jeweils 1 oder 2 - 4 C-Atomen substituiert sein kann und

Ar Biphenyl, Naphthyl oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 oder 2 bis 5 C-Atomen und ferner durch Phenoxy substituiert sein kann und Y eine -CO-, -CH(OH)- oder -CH($OR^3$)-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl, ferner durch

Alkyl oder Alkoxy mit 1 bis 4 C-Atomen substituiert sein kann, oder $R^3$ einen -CO-$R^4$-Rest bedeutet, wobei $R^4$ einen gegebenenfalls durch Halogen, $C_1$-$C_4$-Alkoxy, Oxo (= O) oder Carboxy-$C_1$-$C_4$-alkyl substituierten Alkylrest mit 1 bis 5 C-Atomen oder einen aromatischen Rest bedeutet und

X N oder CH bedeutet, sowie seine für Pflanzen verrägliche Säureadditionssalze und Metallkomplexe.

2.Verbindungen der Formel I, in der $R^1$ Methyl, $R^2$ Wasserstoff oder Methyl, Ar Halogenphenyl und X N bedeutet.

3.Verfahren zur Herstellung von Dioxanyl-azolderivaten der Formel I gemäß Anspruch 1, dadurch gekennzeichnet, daß man

a) ein Azol mit einem alpha-Halogenketon der Formel II

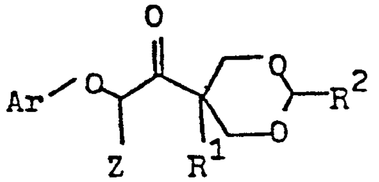

II,

worin $R^1$, $R^2$ und Ar die oben angegebene Bedeutung haben und Z für Chlor oder Brom steht oder

b) einen Alkohol der Formel III

Ar-OH III,

worin Ar die oben angegebene Bedeutung hat, mit einem Keton der Formel IV

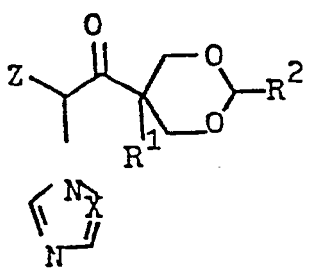

IV,

worin $R^1$, $R^2$, Z und X die oben angegebene Bedeutung haben, umsetzt und die so erhaltenen Verbindungen gegebenenfalls anschließend reduziert und verethert oder verestert und gegebenenfalls eine Säure oder ein Metallsalz addiert.

4. Fungizides Mittel, enthaltend eine Verbindung gemäß Anspruch 1.

5. Fungizides Mittel, enthaltend inerte Zusatzstoffe und ein Dioxanyl-azolderivat der Formel I

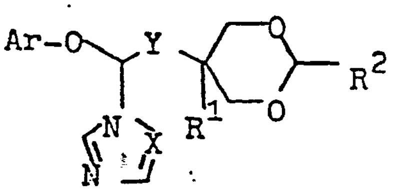

I,

in der

$R^1$ und $R^2$ verschieden oder gleich sind und Wasserstoff oder Alkyl mit 1 bis 5 C-Atomen oder Phenyl bedeuten, wobei der Phenylrest durch Fluor, Chlor, Brom, Nitro, Trifluormethyl, weiterhin durch Alkyl, Alkoxy oder Alkenyl mit jeweils 1 oder 2 - 4 C-Atomen substituiert sein kann und

Ar Biphenyl, Naphthyl oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 oder 2 bis 5 C-Atomen und ferner durch Phenoxy substituiert sein kann und Y eine -CO-, -CH(OH)- oder -CH($OR^3$)-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl, ferner durch Alkyl oder Alkoxy mit 1 bis 4 C-Atomen substituiert sein kann, oder $R^3$ einen -CO-$R^4$-Rest bedeutet, wobei $R^4$ einen gegebenenfalls durch Halogen, $C_1$-$C_4$-Alkoxy, Oxo (= O) oder Carboxy-$C_1$-$C_4$-alkyl substituierten Alkylrest mit 1 bis 5 C-Atomen oder einen aromatischen Rest bedeutet und

X N oder CH bedeutet, sowie seine für Pflanzen verträgliche Säureadditionssalze und Metallkomplexe.

6. Verfahren zur Bekämpfung von Pilzen, dadurch gekennzeichnet, daß man ein Dioxanyl-azolderivat der Formel I

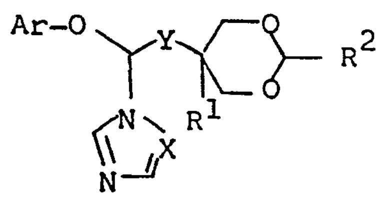

I,

in der

$R^1$ und $R^2$ verschieden oder gleich sind und Wasserstoff oder Alkyl mit 1 bis 5 C-Atomen oder Phenyl bedeuten, wobei der Phenylrest durch Fluor, Chlor, Brom, Nitro, Trifluormethyl, weiterhin durch Alkyl, Alkoxy oder Alkenyl mit jeweils 1 oder 2 - 4 C-Atomen substituiert sein kann und

Ar Biphenyl Naphthyl oder Phenyl bedeutet, wobei der Phenylrest durch Fluor, Chlor, Brom, Iod, Nitro, Trifluormethyl, weiterhin durch Alkyl, Alkoxy oder Alkenyl mit jeweils 1 oder 2 bis 5 C-Atomen und ferner durch Phenoxy substituiert sein kann und Y eine -CO-, -CH(OH)- oder -CH($OR^3$)-Gruppe bedeutet, wobei $R^3$ einen Alkylrest mit 1 bis 8 C-Atomen oder einen gegebenenfalls durch Chlor substituierten Alkenylrest mit 2 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl, ferner durch Alkyl oder Alkoxy mit 1 bis 4 C-Atomen substituiert sein kann, oder $R^3$ einen -CO-$R^4$-Rest bedeutet, wobei $R^4$ einen gegebenenfalls durch

Halogen, $C_1$-$C_4$-Alkoxy, Oxo (= O) oder Carboxy-$C_1$-$C_4$-alkyl substituierten Alkylrest mit 1 bis 5 C-Atomen oder einen aromatischen Rest bedeutet und

X N oder CH bedeutet, sowie seine für Pflanzen verträgliche Säureadditionssalze und Metallkomplexe auf die Pilze oder auf durch Pilzbefall bedrohte Materialien, Flächen, Pflanzen oder Saatgüter einwirken läßt.

7. Verfahren zur Herstellung eines fungiziden Mittels, dadurch gekennzeichnet, daß man eine Verbindung gemäß Anspruch 1 mit einem festen oder flüssigen Trägerstoff mischt.

## Claims

1. A dioxanyl-azole derivative of the formula I

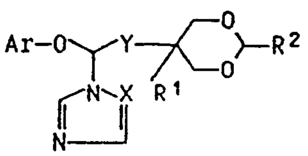

I,

where

$R^1$ and $R^2$ are identical or different and each denotes hydrogen or alkyl of 1 to 5 carbon atoms, or phenyl optionally substituted by fluorine, chlorine, bromine, nitro or trifluoromethyl, or by alkyl, alkoxy or alkenyl each having 1 or 2-4 carbon atoms, and

Ar is biphenyl or naphthyl, or phenyl optionally substituted by fluorine, chlorine, bromine, iodine, nitro or trifluoromethyl, or by alkyl, alkoxy, or alkenyl each having 1 or 2-5 carbon atoms, or by phenoxy, Y is a -CO-, -CH(OH)- or -CH($OR^3$)-group, where $R^3$ is alkyl of 1 to 8 carbon atoms, unsubstituted or chlorine-substituted alkenyl of 2 to 5 carbon atoms or alkynyl of 3 or 4 carbon atoms, or benzyl optionally substituted by fluorine, chlorine, bromine, nitro or trifluoromethyl. or by alkyl or alkoxy each having 1 to 4 carbon atoms, or $R^3$ is -CO-$R^4$, where $R^4$ is alkyl of 1 to 5 carbon atoms optionally substituted by halogen, $C_1$-$C_4$-alkoxy, oxo (=O) or carboxy-$C_1$-$C_4$-alkyl, or an aromatic radical, and

X is N or CH,

and its plant-tolerated acid addition salts and metal complexes.

2. A compound of the formula I, where $R^1$ is methyl, $R^2$ is hydrogen or methyl, Ar is halophenyl, and X is N.

3. A process for the preparation of a dioxanyl-azole derivative of the formula I as claimed in claim 1, wherein

(a) an azole is reacted with an α-halkoketone of the formula II

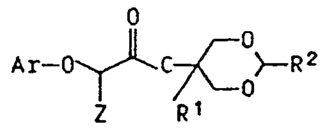

II,

where $R^1$, $R^2$ and Ar have the above meanings, and Z is chlorine or bromine, or

(b) an alcohol of the formula III

Ar - OH III,

where Ar has the above meanings, is reacted with a ketone of the formula IV

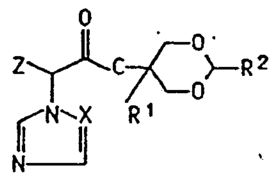

IV,

where $R^1$, $R^2$, Z and X have the above meanings, and the resulting compound is then optionally reduced and etherified or esterified, and, if desired, an acid or metal salt is added.

4. A fungicidal agent containing a compound as claimed in claim 1.

5. A fungidical agent containing inert additives and a dioxanyl-azole derivative of the formula I

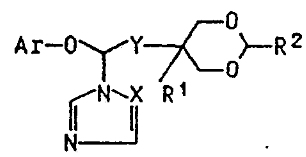

I,

where

$R^1$ and $R^2$ are identical or different and each denotes hydrogen or alkyl of 1 to 5 carbon atoms, or phenyl optionally substituted by fluorine, chlorine, bromine, nitro or trifluoromethyl, or by alkyl, alkoxy or alkenyl each having 1 or 2-4 carbon atoms, and

Ar is biphenyl or naphthyl, or phenyl optionally substituted by fluorine, chlorine, bromine, iodine, nitro or trifluoromethyl, or by alkyl, alkoxy, or alkenyl each having 1 or 2-5 carbon atoms, or by phenoxy, Y is a -CO-, -CH(OH)- or -CH($OR^3$)-group, where $R^3$ is alkyl of 1 to 8 carbon atoms, unsubstituted or chlorine-substituted alkenyl of 2 to 5 carbon atoms or alkynyl of 3 or 4 carbon atoms, or benzyl optionally substituted by fluorine, chlorine, bromine, nitro or trifluoromethyl, or by alkyl or alkoxy each having 1 to 4 carbon atoms, or $R^3$ is -CO-$R^4$, where $R^4$ is alkyl of 1 to 5 carbon atoms optionally substituted by halogen, $C_1$-$C_4$-alkoxy, oxo (=O) or carboxy-$C_1$-$C_4$-alkyl, or an aromatic radical, and

X is N or CH, or a plant-tolerated acid addition salt or metal complex thereof.

6. A method of combating fungi, wherein a dioxanyl-azole derivative of the formula I

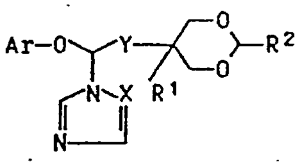

I.

where

$R^1$ and $R^2$ are identical or different and each denotes hydrogen or alkyl of 1 to 5 carbon atoms, or phenyl optionally substituted by fluorine, chlorine, bromine, nitro or trifluoromethyl, or by alkyl, alkoxy or alkenyl each having 1 or 2-4 carbon atoms, and Ar is biphenyl or naphthyl, or phenyl optionally substituted by fluorine, chlorine, bromine, iodine, nitro or trifluoromethyl, or by alkyl, alkoxy, or alkenyl each having 1 or 2-5 carbon atoms, or by phenoxy, Y is a -CO-, -CH(OH)- or -CH($OR^3$)- group, where $R^3$ is alkyl of 1 to 8 carbon atoms, unsubstituted or chlorine-substituted alkenyl of 2 to 5 carbon atoms or alkynyl of 3 or 4 carbon atoms, or benzyl optionally substituted by fluorine, chlorine, bromine, nitro or trifluoromethyl, or by alkyl or alkoxy each having 1 to 4 carbon atoms, or $R^3$ is -CO-$R^4$, where $R^4$ is alkyl of 1 to 5 carbon atoms optionally substituted by halogen, $C_1$-$C_4$-alkoxy, oxo (=O) or carboxy-$C_1$-$C_4$-alkyl, or an aromatic radical, and

X is N or CH, or a plant-tolerated acid addition salt or metal complex thereof is allowed to act on the fungi or on the materials, areas, plants or seed threatened by fungal attack.

7. A process for the production of a fungicidal agent, wherein a compound as claimed in claim 1 is mixed with a solid or liquid carrier.

**Revendications**

1. Dérivé du dioxannyl-azole de la formule I

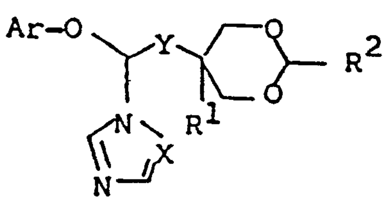

I,

dans laquelle

$R^1$ et $R^2$ sont identiques ou différents et représentent des atomes d'hydrogène ou des radicaux alkyle qui comportent de 1 à 5 atomes de carbone ou des radicaux phényle, où les radicaux phényle peuvent être substitués par des atomes de fluor, de chlore, de brome, des radicaux nitro, trifluorométhyle, mais aussi par des radicaux alkyle, alcoxy ou alcényle comportant chacun 1 ou 2 - 4 atomes de carbone et

Ar représente un radical biphényle, naphtyle ou phényle, où le radical phényle peut être substitué par des atomes de fluor, de chlore, de brome, d'iode, des radicaux nitro, trifluorométhyle, mais aussi par des radicaux alkyle, alcoxy ou alcényle comportant chacun 1 ou 2 à 5 atomes de carbone et peut en outre comporter une substitution phénoxylique et

Y représente un radical -CO-, -CH(OH)- ou -CH($OR^3$)-, où $R^3$ représente un radical alkyle comportant de 1 à 8 atomes de carbone, un radical alcynyle comportant 3 ou 4 atomes de carbone, ou un radical alcényle comportant 2 à 5 atomes de carbone, éventuellement à substitution chlorée, ou un radical benzyle, où le radical benzyle peut être substitué par des atomes de fluor, de chlore, de brome, des radicaux nitro, trifluorométhyle, mais aussi des radicaux alkyle ou alcoxy possédant de 1 à 4 atomes de carbone, ou bien $R^3$ représente un radical -CO-$R^4$- où $R^4$ représente un radical aromatique, ou un radical alkyle comportant de 1 à 5 atomes de carbone, éventuellement substitué par des atomes d'halogènes, des radicaux alcoxy en $C_1$-$C_4$, oxo (= O) ou carboxy-alkyle ($C_1$-$C_4$), et

X représente N ou CH, comme aussi ses sels d'addition d' acides et ses complexes de métaux phytocompatibles.

2. Composés de la formule I dans laquelle $R^1$ représente le radical méthyle, $R^2$ représente un atome d'hydrogène ou le radical méthyle, Ar représente un radical halogénophényle et X représente N.

3. Procédé de préparation de dérivés de dioxannyl-azole de la formule I suivant la revendication 1, caractérisé en ce que

a) On fait réagir un azole sur une alpha-halogénocétone de la formule II

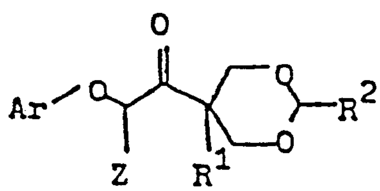

II,

dans laquelle $R^1$, $R^2$ et Ar possèdent les significations qui leur ont été précédemment attribuées et Z représente un atome de chlore ou de brome, ou

b) On fait réagir un alcool de la formule III
Ar-OH III,
dans laquelle Ar possède les significations qui lui ont été précédemment attribuées, sur une cétone de la formule IV

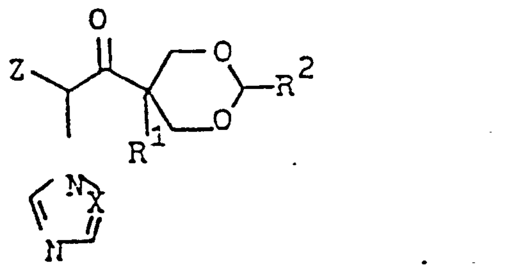

IV,

dans laquelle $R^1$, $R^2$, Z et X possedènt les significations qui leur ont été précédemment attribuées, et on réduit éventuellement ensuite les composés ainsi obtenus et on les éthérifie ou estérifie et on y additionne éventuellement un acide ou un sel de métal.

4. Agent ou composition fongicide contenant un composé suivant la revendication 1.

5. Agent ou composition fongicide, contenant des additifs et un dérivé de dioxannyl-azole de la formule I

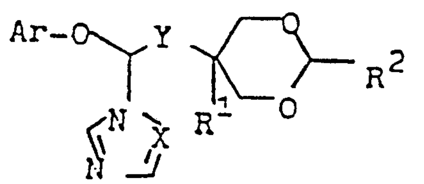

I,

dans laquelle

$R^1$ et $R^2$ sont identiques ou différents et représentent des atomes d'hydrogène ou des radicaux alkyle qui comportent de 1 à 5 atomes de carbone ou des radicaux phényle, où les radicaux phényle peuvent être substitués par des atomes de fluor, de chlore, de brome, des radicaux nitro, trifluorométhyle, mais aussi par des radicaux alkyle, alcoxy ou alcényle comportant chacun 1 ou 2-4 atomes de carbone et

Ar représente un radical biphényle, naphtyle ou phényle, où le radical phényle peut être substitué par des atomes de fluor, de chlore, de brome, d'iode, des radicaux nitro, trifluorométhyle, mais aussi par des radicaux alkyle, alcoxy ou alcényle comportant chacun 1 ou 2 à 5 atomes de carbone et peut en outre comporter une substitution phénoxylique et

Y représente un radical -CO-, -CH(OH)- ou -CH($OR^3$)-, où $R^3$ représente un radical alkyle comportant de 1 à 8 atomes de carbone, un radical alcynyle comportant 3 ou 4 atomes de carbone, ou un radical alcényle comportant 2 à 5 atomes de carbone, éventuellement à substitution chlorée, ou un radical benzyle, où le radical benzyle peut être substitué par des atomes de fluor, de chlore, de brome, des radicaux nitro, trifluorométhyle, mais aussi des radicaux alkyle ou alcoxy possédant de 1 à 4 atomes de carbone, ou bien $R^3$ représente un radical -CO-$R^4$- où $R^4$ représente un radical aromatique, ou un radical alkyle comportant de 1 à 5 atomes de carbone, éventuellement substitué par des atomes d'halogènes, des radicaux alcoxy en $C_1$-$C_4$, oxo (= O) ou carboxy-alkyle ($C_1$-$C_4$), et

X représente N ou CH, comme aussi ses sels d'addition d'acides et ses complexes de métaux phytocompatibles.

6. Procédé pour combattre les champignons, caractérisé en ce que l'on fait agir un dérivé de dioxannyl-azole de la formule I

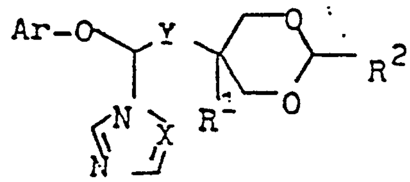

I

dans laquelle

$R^1$ et $R^2$ sont identiques ou différents et représentent des atomes d'hydrogène ou des radicaux alkyle qui comportent de 1 à 5 atomes de carbone ou des radicaux phényle, où les radicaux phényle peuvent être substitués par des atomes de fluor, de chlore, de brome, des radicaux nitro, trifluorométhyle, mais aussi par des radicaux alkyle, alcoxy ou alcényle comportant chacun 1 ou 2-4 atomes de carbone et Ar représente un radical biphényle, naphtyle ou phényle, où le radical phényle peut être substitué par des atomes de fluor, de chlore, de brome, d'iode, des radicaux nitro, trifluorométhyle, mais aussi par des radicaux alkyle, alcoxy ou alcényle comportant chacun 1 ou 2 à 5 atomes de carbone et peut en outre comporter une substitution phénoxylique et

Y représente un radical -CO-, -CH(OH)- ou -CH($OR^3$)-, où $R^3$ représente un radical alkyle comportant de 1 à 8 atomes de carbone, un radical alcynyle comportant 3 ou 4 atomes de carbone, ou un radical alcényle comportant 2 à 5 atomes de carbone, éventuellement à substitution chlorée, ou un radical benzyle, où le radical benzyle peut être substitué par des atomes de fluor, de chlore, de brome, des radicaux nitro, trifluorométhyle, mais aussi des radicaux alkyle ou alcoxy possédant de 1 à 4 atomes de carbone, ou bien $R^3$ représente un radical -CO-$R^4$- où $R^4$ représente un radical aromatique, ou un radical alkyle comportant de 1 à 5 atomes de carbone, éventuellement substitué par des atomes d'halogènes, des radicaux alcoxy en $C_1$-$C_4$, oxo (= O) ou carboxy-alkyle ($C_1$-$C_4$), et

X représente N ou CH, comme aussi ses sels d'addition d'acides et ses complexes de métaux phytocompatibles sur les champignons ou sur les semences plantes, surfaces ou matériaux qui sont les sujets d'attaques par des champignons.

7. Procédé de préparation d'un agent ou d'une composition fongicide, caractérisé en ce que l'on mélange un composé suivant la revendication 1 à un support solide ou liquide.